# FASCICULE DE BREVET EUROPEEN

(11) **EP 1 276 374 B1**
(45) Date de publication et mention de la délivrance du brevet: **17.03.2004**
(21) Numéro de dépôt: 01929723.3
(22) Date de dépôt: 26.04.2001
(51) Int. Cl.: A01N 25/34, A01N 25/10, B65D 47/18, A61F 9/00, A61J 1/14

(54) **PIECE POREUSE ANTI-MICROBIENNE A BASE D'UN MATERIAU POLYMERIQUE GREFFE DE MOTIFS BENZALKONIUM**
PORÖSER ANTIMIKROBIELLER FORMKÖRPER AUF BASIS GEPROPFTEN POLYMERMATERIALS NACH VORBILD DES BENZALKONIUMS
ANTI-MICROBIAL POROUS PART BASED ON POLYMERIC MATERIALS GRAFTED BENZALKONIUM WITH MOTIFS

(30) Priorité: 28.04.2000 FR 0005532
(43) Date de publication de la demande: 22.01.2003
(73) Titulaire: Laboratoire Chauvin S.A., 34009 Montpellier Cédex 1 (FR)
(72) Inventeur: NAJI, Mohammed, 34070 Montpellier (FR); PAGES, Bernard, F-34560 Montbazin (FR); LACOMBE, Jacques, F-34170 Castelnau le Lez (FR)
(74) Mandataire: Bernasconi, Jean
(86) Numéro de dépôt international: PCT/FR2001/001290
(87) Numéro de publication internationale: WO 2001/082696

(56) Documents cités:
- EP-A- 0 478 445
- EP-A- 0 558 357
- EP-A- 0 952 168
- WO-A-95/17152
- FR-A- 2 686 610
- DATABASE WPI Section Ch, Week 199607 Derwent Publications Ltd., London, GB; Class A13, AN 1996-064967 XP002157636 & JP 07 323206 A (EBARA CORP), 12 décembre 1995 (1995-12-12)

## Description

La présente invention concerne le domaine du conditionnement de solutions liquides auxquelles on souhaite garantir une non-contamination microbienne. Les domaines plus particulièrement visés dans le cadre de la présente invention sont ceux de la pharmacie et notamment celui de l'ophtalmologie.

D'une manière générale, toute solution liquide relevant du domaine thérapeutique et notamment ophtalmique, est conditionnée dans un dispositif de type flacon adapté d'une buse pour la distribution de cette solution. Cette buse est jusqu'à son usage, close hermétiquement de manière à protéger la solution conditionnée contre tout risque de contamination, en particulier par des germes de l'atmosphère ambiante.

Le problème posé et résolu selon la présente invention vise la garantie de cette protection à la solution conditionnée après ouverture du flacon et pendant sa période d' utilisation.

Aujourd'hui, on dispose de deux alternatives pour assurer à l'usager cette protection anti-microbienne.

La première option consiste à lui proposer des solutions liquides dans lesquelles ont été incorporés des conservateurs anti-microbiens ou bactéricides. Ce type de solution a l'avantage d'être compatible avec un usage prolongé de la solution conditionnée dans le temps. En revanche, ces conservateurs peuvent être à l'origine chez certains usagers d'effets secondaires indésirables.

La seconde option consiste à formuler la solution sous la forme de doses à usage unique. Les solutions ainsi conditionnées sont dépourvues en conservateurs mais en revanche incompatibles avec un usage prolongé au-delà de 3 heures. L'inconvénient majeur de ce second type de conditionnement est donc triple: coût, encombrement du conditionnement (pour un mois de traitement) et difficulté d'instillation et impossibilité de réutilisation sans risque de contamination.

Actuellement, il existe donc un besoin d'un nouveau conditionnement pour solution liquide stérile qui soit compatible avec un usage prolongé dans le temps de cette solution, mais qui ne nécessite pas l'incorporation dans ladite solution de conservateurs anti-microbiens et/ou bactéricides.

La demande de brevet WO 95/17 512, décrit un flacon muitidoses de conditionnement d'une solution stérile, comprenant un embout de distribution constitué d'un canal équipé par au moins un filtre poreux (0,2 à 1,2 microns), qui peut être de nature polymérique, sur lequel est greffée en surface une combinaison de matériau métallique antimicrobien et entre autres de polymères contenant des dérivés benzalkonium, immobilisés par réticulation.

Dans un tout autre domaine, la demande de brevet JP 07-323206 décrit un filtre à air constitué d'un empilement de deux types de non-tissés de fibres échangeuses d'ions. Les fibres échangeuses d'ions en polypropylène et polyéthylène sont irradiées, puis greffées en surface par du chlorométhyl styrène (CMS) ou du 4-vinyl pyridine (4VP) respectivement, puis trempées dans une solution d'amine pour générer à la surface des fibres, un polymère réticulé à base d'ammonium quaternaire.

De nouveaux types de dispositifs ont été élaborés en prenant en compte ces considérations. Toutefois, ils ne sont pas jugés totalement satisfaisants dans la mesure où ils impliquent généralement un nouveau concept de flaconnage qui, pour des raisons de coût, ne fait pas l'adhésion des industriels.

La présente invention a précisément pour objet de proposer une solution permettant de donner satisfaction à l'ensemble des points discutés ci-dessus.

Un premier aspect de l'invention concerne donc une pièce poreuse anti-microbienne à base d'un matériau polymérique greffé en surface et en masse de motifs ammoniums à activité anti-microbienne et/ou bactéricide et/ou fongicide, caractérisée en ce qu'elle possède une porosité d'une part suffisamment élevée pour permettre le passage, à travers sa structure, d'un liquide de viscosité compatible et d'autre part suffisamment faible pour assurer le piégeage, au sein de cette structure, de germes contaminants.

Les inventeurs ont ainsi mis en évidence qu'il était possible à l'aide d'une telle pièce poreuse de répondre à l'ensemble des exigences formulées ci-dessus.

La pièce poreuse selon l'invention a pour premier avantage d'être totalement compatible avec les dispositifs de flaconnage déjà existants. Compte tenu de sa taille, elle peut être insérée facilement dans la buse de distribution de flacons et assurer à ce niveau, une fonction de barrage efficace entre la solution conditionnée dans le réceptacle du flacon et l'extrémité de la buse de distribution exposée à l'atmosphère ambiante.

De par sa structure chimique, qui incorpore en surface et en masse des motifs à activité anti-microbienne et/ou bactéricide, elle permet, par un effet de rétention des germes contaminants, au sein de sa surface active, un isolement efficace de ceux-ci vis-à-vis de la solution liquide, puis, par contact intime entre le matériau et les germes, la réduction et l'éradication dans le temps de tels micro-organimes.

Enfin, sa structure poreuse qui présente en profondeur un réseau interne de canalicules, autorise le passage de la solution liquide au moment de son administration.

Le triple objectif exposé précédemment est par conséquent atteint à l'aide de la pièce poreuse selon l'invention.

Plus précisément, la structure chimique de cette pièce poreuse dérive d'un polymère constitué de motifs de formule générale I :

-[CH₂-CH(Ar)]- (I)

dans laquelle Ar représente une chaîne de formules :

-Ph-CH₂-N^{⊖}(CH₃)₂R, X^{⊖} (II)

ou

-Ph-CH₂-X (III)

dans lesquelles :
- R représente un alkyle en C₂-C₁₅,
- Ph un motif phényle et
- X⁻ est choisi parmi les anions habituellement utilisés dans le domaine et pharmaceutiquement acceptables avec des chaînes de formule II étant présentes en quantité suffisante pour conférer audit matériau des propriétés anti-microbiennes.

A titre représentatif des ions figurés par X⁻, on peut plus particulièrement citer les halogénures comme les chlorure, bromure, iodure, les acétate, benzoate, carbonate, citrate, formate, gluconate, glycolate, hydroxyde, lactate, malate, maléate, malonate, nitrate, phosphate, propionate, succinate, sulfate, tartrate et analogues. Selon une variante préférée de l'invention, il s'agit d'un anion chlorure.

Selon une variante préférée de l'invention, le polymère dérive d'un polystyrène, de préférence non réticulé.

En ce qui concerne les motifs benzalkoniums de formule II, ils comportent de préférence à titre d'ammonium quaternaire un groupement diméthyltétradécyle ammonium.

Le taux en motifs de type benzalkonium de formule II présent sur la structure polymérique est susceptible de varier en fonction notamment de la nature du squelette polymérique et de celle des substituants figurant sur le motif benzalkonium.

En fait, il est ajusté de manière à atteindre un compromis entre l'efficacité anti-microbienne et/ou anti-bactérienne et, la garantie, au niveau du matériau plastique ainsi constitué, d'une hydrophobie compatible avec l'usage recherché. En effet, un taux de greffage trop élevé au niveau du polymère peut conférer à celui-ci un caractère trop hydrophile qui se traduit par la formation d'un gel lorsque le matériau entre en contact avec une solution aqueuse, phénomène non compatible avec l'usage recherché.

Lorsque le polymère est constitué d'un squelette polystyrène, ce compromis est avantageusement atteint pour un pourcentage molaire de greffage d'environ 15 à 30%, et de préférence d'environ 20%, soit un pourcentage pondéral en motifs benzalkonium de formule II d'environ 30 à 40%, et de préférence d'environ 36%, de la masse du polymère greffé ainsi obtenu. II est bien entendu possible d'abaisser cette valeur en fonction de l'efficacité anti-microbienne recherchée.

Préférentiellement, le matériau polymérique est un polystyrène greffé en partie de motifs diméthyltétradécylammonium et de préférence, greffé à un pourcentage molaire compris entre 18 et 25%.

Le matériau polymérique peut être obtenu en faisant réagir un polymère constitué de motifs 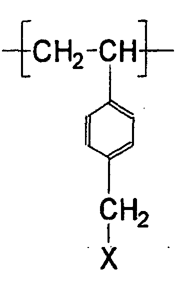 tel que de préférence un polystyrène chlorométhylé avec une amine de formule N(CH₃)₂(R) avec X et R étant tels que définis précédemment, de préférence en milieu homogène, c'est-à-dire sous une forme solubilisée, et dans un rapport molaire ajusté en fonction du taux de greffage recherché.

Selon un mode de préparation privilégié, le matériau polymérique est préalablement purifié par dissolution dans un solvant organique type dichlorométhane et reprécipitation par addition d'un alcool de type méthanol. Le polymère ainsi recristallisé est recueilli par filtration. Cette étape permet d'éliminer du polymère les composés de faibles poids moléculaires qui, après la réaction de greffage, sont susceptibles d'être relargués en milieu aqueux.

Le polymère ainsi purifié et l'amine précédemment définie sont solubilisés en milieu organique type acétone et maintenus sous agitation constante pendant 24 heures à température ambiante.

De préférence, l'amine greffée est la diméthyltétradécylamine et le pourcentage de greffage molaire est voisin de 20%.

Le matériau ainsi obtenu est ensuite isolé de préférence par précipitation en incorporant au milieu réactionnel un solvant type hexane. Ce traitement permet non seulement la purification du matériau mais l'obtention d'une poudre de granulométrie homogène. L'ajustement des conditions d'agitation et des volumes de solvant mis en oeuvre conduit à un produit de pureté et de granulométrie parfaitement maîtrisées.

L'activité anti-microbienne et/ou bactéricide et/ou fongicide du matériau ainsi obtenu a été testée notamment vis-à-vis de souches Pseudomonas, Staphylococcus, Candida, Aspergillus. Ce test fait l'objet de l'exemple 2 ci-après.

L'activité anti-microbienne du matériau polymérique satisfait aux critères A de la Pharmacopée Européenne pour 3 germes de référence (Staphylococcus aureus, Pseudomonas aeruginosa, Candida albicans) et aux critères B pour le quatrième germe de référence (Aspergillus niger) réputé pour être peu sensible aux ammoniums quaternaires même solubilisés. Avec une charge contaminante initiale de 100.000 germes (sous 0,1 ml de suspension microbienne) pour 100 mg de matériau, ces résultats obtenus sans agitation sont tout à fait satisfaisants et reproductibles.

De même, l'insolubilité aqueuse du matériau et l'absence de relargage de ses motifs constitutifs (oligomères ammonium quaternaire de faible poids moléculaire) ont été démontrées après 24 heures de contact sous forte agitation entre 2 grammes de matériau et 100 ml d'eau distillée : l'intégrité des solutions est donc assurée à long terme, puisque le contact entre la pièce poreuse et la solution conditionnée est limité au passage à travers la pièce poreuse lors de la délivrance.

Le matériau polymérique ainsi obtenu est ensuite façonné en une pièce poreuse. Ce façonnage peut être réalisé selon différentes techniques familières à l'homme de l'art telle que, par exemple, celle qui consiste à compacter la poudre du matériau précédemment défini sous forme d'un fritté. Dans un moule de dimensions définies et dans des conditions paramétrées de température et de pression, les grains de matériau sont ramollis pour s'accoler entre eux de manière à former ainsi une pièce plastique rigide. Les interstices laissés libres entre les grains de poudre accolés créent un réseau multidimensionnel de canalicules qui confèrent à la pièce une porosité en profondeur.

A l'issue de ce façonnage, on obtient donc un matériau fonctionnalisé en surface et en masse par des motifs à activité anti-microbienne et possédant une porosité suffisante pour permettre le passage à travers la structure d'une solution de faible viscosité.

La porosité en profondeur de la pièce précédemment définie est conditionnée d'une part par l'homogénéité de granulométrie du matériau et la taille moyenne des grains de matériau, d'autre part par la tenue de ce même matériau aux conditions de température et de pression mises en oeuvre.

Préférentiellement, la pièce poreuse selon l'invention se présente sous forme d'un fritté dont la porosité en profondeur est comprise entre 5 et 30 microns, nécessitant une granulométrie moyenne du matériau comprise entre 30 et 350 microns et préférentiellement entre 80 et 125 microns.

Un troisième aspect de l'invention concerne l'utilisation de la pièce poreuse revendiquée dans la buse de distribution d'un flacon de conditionnement d'une solution liquide.

Les solutions concernées peuvent être de natures très diverses. Il peut notamment s'agir de solutions à usage alimentaire (de type jus de fruits, yaourts liquides), à usage médicamenteux (tels des solutions buvables, sirops, solutions ophtalmiques, solutions nasales, solutions auriculaires), ou encore à usage dermatologique (tel que des fluides corporels, des solutions démaquillantes).

En fait, le seul critère de sélection de cette solution est lié à sa viscosité. Elle doit bien entendu être compatible avec la porosité en profondeur de la pièce poreuse.

Selon un mode préféré de l'invention, la solution conditionnée est une solution ophtalmique.

Un troisième aspect de l'invention concerne plus particulièrement une buse de distribution d'une solution liquide, du type comprenant un tube comportant un canal interne et muni, à l'une de ses extrémités, d'un embout d'emboîtement dans une ouverture d'un flacon et, à l'autre desdites extrémités, d'un orifice d'écoulement goutte-à-goutte de la solution liquide, caractérisé en ce que ie tube comporte dans ledit canal interne une pièce poreuse telle que définie précédemment.

Comme discuté précédemment, un des avantages de la pièce poreuse revendiquée est de pouvoir être adaptable à tout système de flaconnage déjà existant. II est en effet possible de façonner, à partir du matériau polymérique, des pièces poreuses de tailles ajustées aux buses de distribution des flacons considérés.

Les exemples et figures sont présentés ci-après à titre illustratif de la présente invention.

### Figures

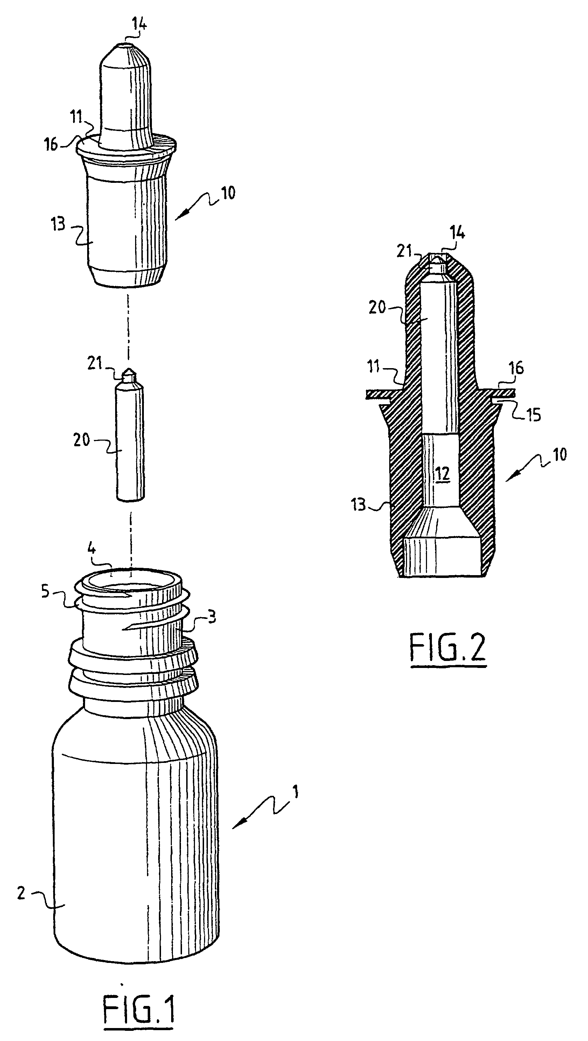

La figure 1 représente une vue schématique en perspective éclatée d'un flacon équipé d'une buse de distribution de la solution liquide et la figure 2 représente une vue schématique en coupe axiale de cette buse munie d'une pièce poreuse anti-microbienne conforme à l'invention.

Sur la figure 1, on a représenté un flacon désigné dans son ensemble par la référence 1 et qui comporte un corps 2 formant un réservoir destiné à contenir la solution liquide. Le corps 2 comporte à sa partie supérieure un goulot 3 muni d'une ouverture 4 pour le montage d'une buse de distribution de ladite solution liquide contenue dans le corps 2.

Par ailleurs, le col 3 est pourvu sur sa surface externe d'un filetage 5 pour le vissage d'un capuchon, non représenté, destiné à obturer la buse.

La buse désignée par la référence générale 10 est formée par un tube 11 comportant un canal interne 12. Ce tube 11 est muni, à l'une de ses extrémités, d'un embout 13 d'emboîtement dans l'ouverture 4 du corps 2 du flacon 1 et, à l'autre desdites extrémités, d'un orifice 14 d'écoulement goutte-à-goutte de la solution liquide contenue dans ledit flacon 1.

Le tube 11 est également muni extérieurement d'une rainure 15 d'encliquetage sur un rebord ménagé au niveau de l'orifice 4 du corps 2 et également d'une collerette 16 d'appui sur le bord supérieur du col 3 du flacon 1.

Le tube 11 comporte dans le canal interne 12 une pièce poreuse anti-microbienne 20.

De préférence, la pièce poreuse 20 comporte à l'une de ses extrémités une pointe 21 destinée à pénétrer dans l'orifice 14 d'écoulement de la solution liquide.

Cette pièce poreuse 20 possède une porosité d'une part suffisamment élevée pour permettre le passage, à travers sa structure, d'un liquide de viscosité compatible et d'autre part suffisamment faible pour assurer le piégeage, au sein de cette structure, de germes contaminants.

Ces germes y sont ensuite, par activité de contact, réduits et éradiqués.

### EXEMPLES

### EXEMPLE 1 : Fabrication d'un matériau anti-microbien.

Le polystyrène chlorométhylé correspond au réactif Aldrich ref : 18,253-2/lot 00128 TQ.

La diméthyltétradécylamine correspond au réactif Fluka ref :41653 / lot 363679/1-22399.

Le polystyrène chlorométhylé (21 g) est dispersé dans 2000 ml de méthanol pendant 1 heure, récupéré par filtration, dissous dans 130 ml de dichlorométhane puis reprécipité dans 2000 ml de méthanol. Cette étape est répétée. Le polymère ainsi purifié est isolé par filtration après 1 h 30 d'agitation puis séché à l'étuve 40°C.

Dans un ballon de 2 litres, on solubilise 20 g de polymère purifié dans 40 ml d'acétone sous agitation puis on incorpore 6,4 g de diméthyltétradécylamine. L'ensemble est laissé sous agitation pendant 24 heures.

Le produit greffé est isolé par précipitation en ajoutant progressivement dans le milieu réactionnel sous agitation 1500 ml d'hexane. L'ensemble est agité pendant 4 heures puis le produit obtenu est séché à l'étuve 40°C.

On tamise le produit obtenu de façon à obtenir une granulométrie comprise entre 80 et 125 microns.

25 grammes du produit ainsi obtenu sont lavés dans 1000 ml d'eau distillée pendant 1 heure sous agitation et le produit final est isolé par filtration avant séchage à l'étuve 40°C pendant 12 heures. On obtient ainsi 25 grammes du matériau attendu.

La caractérisation du matériau obtenu a été réalisée par Résonance Magnétique Nucléaire du proton dans le chloroforme deutérié, Chromatographie de Perméation de Gel, dosage des chlorures et Analyse Thermique Différentielle : les résultats confirment la structure attendue du polymère.

L'insolubilité aqueuse du matériau a été vérifiée après 24 heures de contact sous forte agitation entre 2 grammes de matériau et 100 ml d'eau distillée : aucune perte de poids significative n'est décelée sur le matériau avant et après traitement et la solution aqueuse ne contient pas de motifs benzalkoniums solubles. Les contrôles UV, CLHP et CPG de cette même solution permettent de conclure que les composés de faible poids moléculaire constitutifs du polymère ne sont pas relargués dans l'eau à une dose supérieure à 0,05 mg/litre, confirmant ainsi que l'activité anti-microbienne du matériau est une activité de contact.

### EXEMPLE 2 : Efficacité anti-microbienne de la pièce poreuse.

Afin de vérifier l'activité anti-microbienne du matériau précédemment défini, le produit est soumis au test d'efficacité de la conservation anti-microbienne de la Pharmacopée Européenne.

Les germes tests sont Pseudomonas aeruginosa, Staphylococcus aureus, Candida albicans, Aspergillus niger.

Pour chaque germe testé, 4 tubes à hémolyse contenant chacun 100 mg de matériau sont inoculés avec 100.000 micro-organismes sous 0,1 ml; homogénéiser et laisser en contact sans agitation.

A chaque échéance prescrite (6 heures, 24 heures, 7 jours, 14 jours, 28 jours de contact), on procède à la numération des germes viables résiduels.

Les résultats obtenus sont exprimés par rapport à la numération initiale de l'inoculum sous forme de réduction logarithmique décimale.

Les critères de la Pharmacopée Européenne sont donnés dans le tableau suivant :

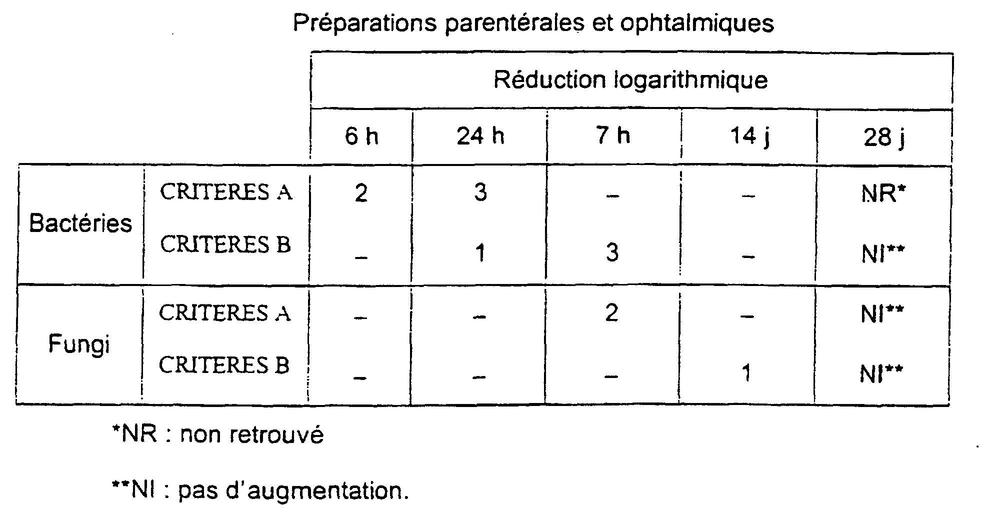

Les résultats obtenus sur le matériau selon l'invention précédemment décrit sont listés dans le tableau ci-après :

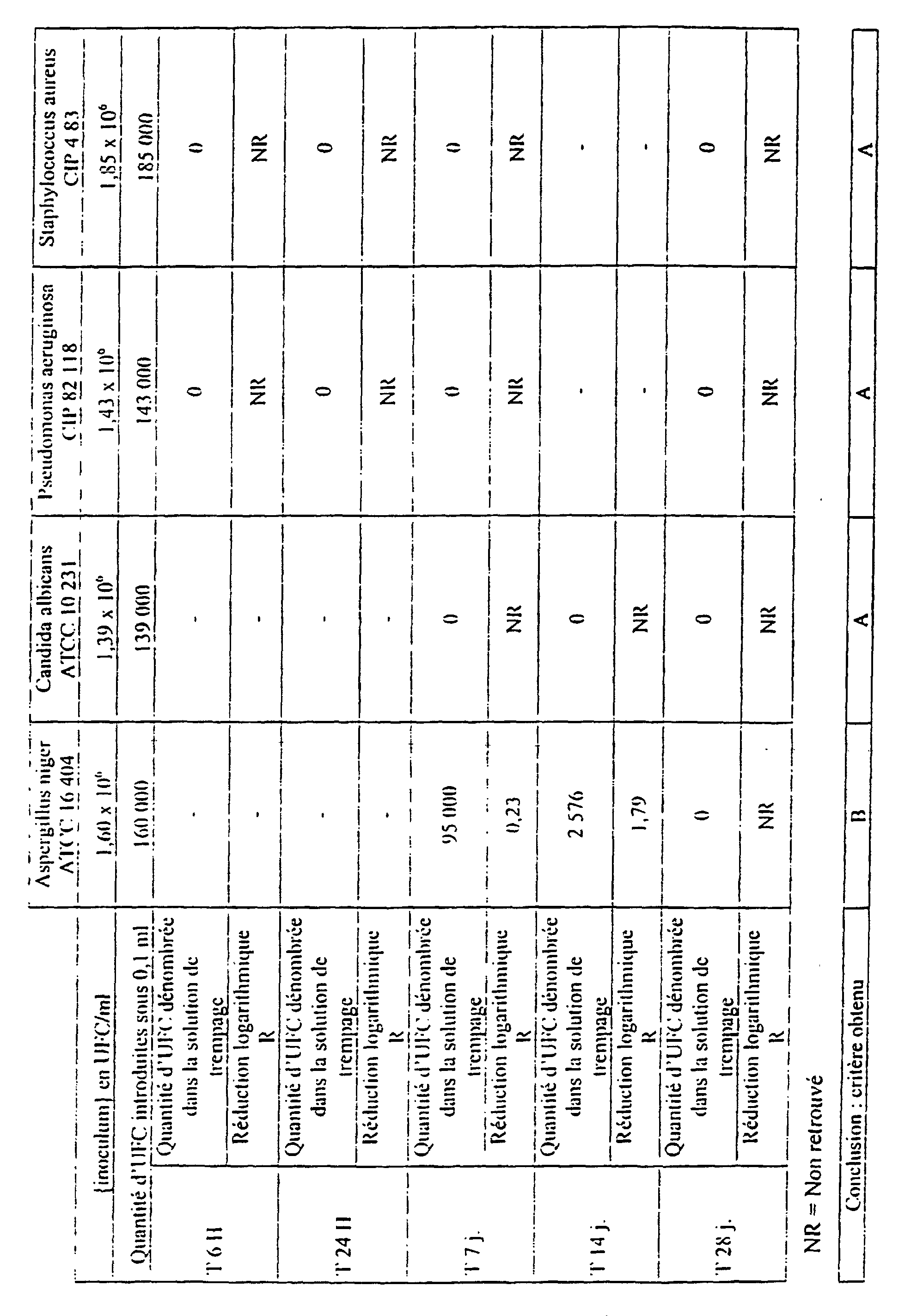

L'activité anti-microbienne du matériau polymérique satisfait au critères A de la pharmacopée européenne pour Staphylococcus aureus, Pseudomonas aeruginosa, Candida albicans (les germes étant totalement éradiqués après respectivement 6 heures pour les deux bactéries et 7 jours pour la levure) et aux critères B pour Aspergillus niger (la moisissure étant totalement éradiquée après 28 jours).

## Revendications

1. Pièce poreuse antimicrobienne à base d'un matériau polymérique dérivé d'un polystyrène, greffé en surface et en masse de motifs ammoniums à activité anti-microbienne et/ou bactéricide et/ou fongicide, **caractérisée en ce qu'**elle possède une porosité en profondeur comprise entre 5 et 30 microns.

2. Pièce poreuse selon la revendication 1, **caractérisée en ce que** le matériau polymérique dérive d'un polymère comprenant des motifs de formule (I) :
-[CH₂-CH(Ar)]- (I)
dans laquelle Ar représente une chaîne de formules :
-Ph-CH₂-N^{⊖}(CH₃)₂R, X^{⊖} (II)
ou
-Ph-CH₂-X (III)
dans lesquelles :
- R représente un alkyle en C₂-C₁₅,
- Ph un motif phényle et
- l'ion X⁻ est un anion pharmaceutiquement acceptable, avec des chaînes de formule II, le matériau polymérique étant greffé à un pourcentage molaire de 15 à 30%.

3. Pièce poreuse selon la revendication 2, **caractérisée en ce que** le polymère est un polystyrène greffé en partie de motifs diméthyltétradécylamine.

4. Pièce poreuse selon la revendication 3, **caractérisée en ce que** le polystyrène est greffé à un pourcentage pondéral en motifs benzalkonium de 30 à 40% de la masse du polymère greffé.

5. Pièce poreuse selon l'une des revendications précédentes, **caractérisée en ce qu'**elle se présente sous la forme d'un fritté.

6. Procédé de préparation d'une pièce poreuse selon l'une des revendications précédentes, **caractérisé en ce que** :
- on fait réagir un polymère constitué de motifs 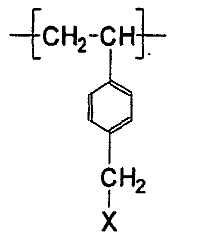 avec une amine de formule N(CH₃)₂(R) avec X et R étant tels que définis en revendication 2, et dans un rapport molaire ajusté en fonction du taux de greffage recherché,
- on isole ledit matériau greffé par précipitation, et
- on façonne la poudre ainsi obtenue sous la forme de ladite pièce poreuse.

7. Procédé de préparation selon la revendication 6, **caractérisé en ce que** la réaction a lieu en milieu homogène.

8. Procédé de préparation selon la revendication 6 ou 7, **caractérisé en ce que** le polymère de départ est un polystyrène chlorométhylé.

9. Procédé selon l'une des revendications 6 à 8, **caractérisé en ce que** l'amine greffée est la diméthyltétradécylamine et **en ce que** le pourcentage molaire de greffage est voisin de 20%.

10. Procédé selon l'une des revendications 6 à 9, **caractérisé en ce que** le façonnage de la poudre en une pièce poreuse est réalisé à l'aide d'une technique de frittage.

11. Procédé selon l'une des revendications 6 à 10, **caractérisé en ce que** le matériau polymérique possède une granulométrie comprise entre 30 et 350 microns.

12. Procédé selon la revendication 11, **caractérisé en ce que** le matériau polymérique possède une granulométrie comprise entre 80 et 125 microns.

13. Utilisation d'une pièce poreuse selon l'une des revendications 1 à 5 dans la buse de distribution d'un flacon de conditionnement d'une solution liquide.

14. Utilisation selon la revendication 13, **caractérisée en ce que** la solution liquide est une solution à usage alimentaire, médicamenteux, ou dermatologique.

15. Utilisation selon la revendication 13 ou 14, **caractérisée en ce qu'**il s'agit d'un flacon de conditionnement d'une solution ophtalmique.

16. Buse de distribution d'une solution liquide, du type comprenant un tube comportant un canal interne et muni, à l'une de ses extrémités, d'un embout d'emboîtement dans une ouverture d'un flacon et, à l'autre desdites extrémités, d'un orifice d'écoulement goutte-à-goutte de la solution liquide, **caractérisée en ce que** le tube comporte dans ledit canal interne une pièce poreuse selon l'une des revendications 1 à 5.

## Patentansprüche

1. Antimikrobielles poröses Formteil auf der Basis eines Polymermaterials, das von einem Polystyrol stammt und an der Oberfläche und in der Masse mit Ammonium-Einheiten gepfropft ist und eine antimikrobielle und/oder bakterizide und/oder fungizide Aktivität aufweist, **dadurch gekennzeichnet, dass** es eine Tiefenporosität zwischen 5 und 30 Mikron besitzt.

2. Poröses Formteil nach Anspruch 1, **dadurch gekennzeichnet, dass** das Polymermaterial von einem Polymer stammt, das Einheiten der Formel (I) umfasst:
-[CH₂-CH(Ar)]- (I)
worin Ar eine Kette der Formeln:
-Ph-CH₂-N^{⊖}(CH₃)₂R, X^{⊖} (II)
oder
-Ph-CH₂-X (III)
darstellt, in denen:
- R ein C₂-C₁₅-Alkyl darstellt,
- Ph ein eine Phenyleinheit darstellt und
- das Ion X⁻ ein pharmazeutisch verträgliches Anion mit den Ketten der Formel II ist, wobei das Polymermaterial in einem Molprozentsatz von 15 bis 30% gepfropft ist.

3. Poröses Formteil nach Anspruch 2, **dadurch gekennzeichnet, dass** das Polymer ein Polystyrol ist, das teilweise mit Dimethyltetradecylamin-Einheiten gepfropft ist.

4. Poröses Formteil nach Anspruch 3, **dadurch gekennzeichnet, dass** das Polystyrol in einem Gewichtsprozentsatz von 30 bis 40%, bezogen auf die Masse des gepfropften Polymers, mit Benzalkonium-Einheiten gepfropft ist.

5. Poröses Formteil nach einem der vorhergehenden Ansprüche, **dadurch gekennzeichnet, dass** es die Form eines Sinterteils hat.

6. Verfahren zur Herstellung eines porösen Formteils nach einem der vorhergehenden Ansprüche, **dadurch gekennzeichnet, dass**:
- man ein Polymer, das aus den Einheiten besteht 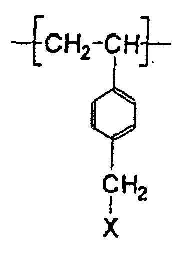 mit einem Amin der Formel N(CH₃)₂R, wobei X und R wie im Anspruch 2 definiert sind, und in einem Molverhältnis, das in Abhängigkeit von dem gewünschten Pfropfungsgrad eingestellt ist, umsetzt,
- das gepfropfte Material durch Fällung isoliert und
- das erhaltene Pulver in die Form des porösen Formteils formt.

7. Verfahren zur Herstellung nach Anspruch 6, **dadurch gekennzeichnet, dass** die Umsetzung in einem homogenen Medium stattfindet.

8. Verfahren zur Herstellung nach Anspruch 6 oder 7, **dadurch gekennzeichnet, dass** das Ausgangspolymer ein chlormethyliertes Polystyrol ist.

9. Verfahren nach einem der Ansprüche 6 bis 8, **dadurch gekennzeichnet, dass** das gepfropfte Amin Dimethyltetradecylamin ist und dass der Molprozentsatz der Pfropfung nahe bei 20% liegt.

10. Verfahren nach einem der Ansprüche 6 bis 9, **dadurch gekennzeichnet, dass** das Formen des Pulvers in ein poröses Formteil mithilfe einer Sintertechnik durchgeführt wird.

11. Verfahren nach einem der Ansprüche 6 bis 10, **dadurch gekennzeichnet, dass** das Polymermaterial eine Granulometrie zwischen 30 und 350 Mikron besitzt.

12. Verfahren nach Anspruch 11, **dadurch gekennzeichnet, dass** das Polymermaterial eine Granulometrie zwischen 80 und 125 Mikron besitzt.

13. Verwendung eines porösen Formteils nach einem der Ansprüche 1 bis 5 in der Verteilungsdüse einer Flasche zur Behandlung einer flüssigen Lösung.

14. Verwendung nach Anspruch 13, **dadurch gekennzeichnet, dass** die flüssige Lösung eine Lösung zur Nahrungs-, Arzneimittel- oder dermatologischen Verwendung ist.

15. Verwendung nach Anspruch 13 oder 14, **dadurch gekennzeichnet, dass** es sich um eine Flasche zur Behandlung einer ophthalmologischen Lösung handelt.

16. Düse zur Verteilung einer flüssigen Lösung des Typs, der eine Röhre umfasst, die einen inneren Kanal enthält und an einem ihrer Enden mit einem Ansatzstück zum Einstecken in eine Öffnung einer Flasche und an dem anderen Ende mit einer Öffnung zum tropfenweisen Entnehmen der flüssigen Lösung ausgestattet ist, **dadurch gekennzeichnet, dass** die Röhre in dem inneren Kanal ein poröses Formteil nach einem der Ansprüche 1 bis 5 besitzt.

## Claims

1. Antimicrobial porous component based on a polymeric material derived from polystyrene, grafted on the surface and throughout with ammonium units having antimicrobial and/or bactericidal and/or fungicidal activity, **characterized in that** it possesses a porosity between 5 and 30 microns deep.

2. Porous component according to claim 1, **characterized in that** the polymeric material derived from a polymer including units of formula (I) :
-[CH₂-CH(Ar)]- (I)
in which Ar represents a chain with the formulae :
-Ph-CH₂-N^{⊖}(CH₃)₂R, X^{⊖} (II)
or
-Ph-CH₂-X (III)
in which:
- R represents an alkyl with C₂-C₁₅,
- Ph is a phenyl unit and
- - the X⁻ ion is a pharmaceutically acceptable anion, with the chains of formula II, the polymeric material being grafted with a molar percentage of 15 to 30 %.

3. Porous component according to claim 2, **characterized in that** the polymer is a polystyrene partly grafted with dimethyltetradecylamine units.

4. Porous component according to claim 3, **characterized in that** the polystyrene is grafted with a weight percentage in benzalkonium units of 30 to 40 % of the grafted polymer.

5. Porous component according to one of the preceding claims, **characterized in that** it is in the form of a sinter.

6. Method for preparing a porous component according to one of the preceding claims, **characterized in that**:
- a polymer consisting of 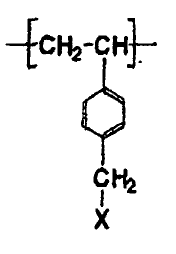 units is reacted with an amine of formula N(CH₃)₂R with X and R being as defined in claim 2, and in a molar ratio adjusted as a function of the desired degree of grafting,
- the said grafted material is isolated by precipitation, and
- the powder obtained in this way is fashioned into the said porous component.

7. Preparative method according to claim 6, **characterised in that** the reaction takes place in a homogeneous medium.

8. Preparative method according to claims 6 or 7, **characterised in that** the starting polymer is a chloromethylated polystyrene.

9. Method according to one of claims 6 to 8, **characterised in that** the grafted amine is dimethyltetradecylamine and **in that** the molar percentage of grafting approaches 20 %.

10. Method according to one of claims 6 to 9, **characterised in that** fashioning of the powder into a porous component is carried out with the aid of a sintering technique.

11. Method according to one of claims 6 to 10, **characterised in that** the polymeric material possesses a particle size of between 30 and 350 microns.

12. Method according to claim 11, **characterised in that** the polymeric material possesses a particle size of between 80 and 125 microns.

13. Use of a porous component according to one of claims 1 to 5 in the distribution nozzle of a bottle for packaging a liquid solution.

14. Use according to claim 13, **characterised in that** the liquid solution is a solution for food, medicinal or dermatological use.

15. Use according to claim 13 or 14, **characterised in that** it concerns a bottle for packaging an ophthalmic solution.

16. Nozzle for distributing a liquid solution, of the type comprising a tube having an internal channel and provided, at one of its ends, with an end piece fitting into an opening of a bottle, and, at the other of the said ends, an orifice for allowing the liquid solution to flow out drop-by-drop, **characterised in that** the tube includes, in the said internal channel, a porous component according to one of claims 1 to 5.
